# FASCICULE DE BREVET EUROPEEN

(11) **EP 1 755 476 B1**
(45) Date de publication et mention de la délivrance du brevet: **16.11.2011**
(21) Numéro de dépôt: 05778724.4
(22) Date de dépôt: 16.06.2005
(51) Int. Cl.: A61C 3/03, A61C 17/20

(54) **INSERT POUR APPAREIL DENTAIRE A ULTRASONS**
EINSATZ FÜR EINE ULTRASCHALL-FRÄSBOHREINHEIT
INSERT FOR ULTRASONIC BURR-DRILL UNIT

(30) Priorité: 18.06.2004 FR 0406630
(43) Date de publication de la demande: 28.02.2007
(73) Titulaire: SOCIETE POUR LA CONCEPTION DES APPLICATIONS DES TECHNIQUES ELECTRONIQUES, 33708 Merignac Cedex (FR)
(72) Inventeur: DOUSSIN, Jean-Claude, F-33140 Villenave D'Ornon (FR); GANGNEUX, Philippe, F-33450 Saint Loubes (FR); RICHER, Jean-Michel, F-33127 Martignas (FR)
(74) Mandataire: Desormiere, Pierre-Louis
(86) Numéro de dépôt international: PCT/FR2005/001508
(87) Numéro de publication internationale: WO 2005/102204

(56) Documents cités:
- US-A- 3 990 452
- US-A- 4 984 985
- US-A1- 2003 108 844
- US-B1- 6 193 515
- US-B1- 6 328 566

## Description

### Arrière plan de l'invention

La présente invention concerne les appareils de traitement dentaire et plus particulièrement les appareils à ultrasons, tels que les appareils de détartrage, qui comprennent des instruments vibrant à des fréquences ultrasonores.

Ce type d'appareil est formé essentiellement d'une pièce à main comprenant un transducteur accouplé mécaniquement à un instrument vibrant dénommé « insert » ou « sonotrode », la pièce à main étant reliée à un générateur d'ultrasons.

L'insert est une pièce interchangeable qui présente une grande variété de formes suivant le traitement auquel elle est destinée. Des exemples de tels inserts sont décrits notamment dans les documents US 6 312 256, US 4 283 175 et US 6 328 566. L'amplitude ou la puissance des ondes ultrasonores transmises par le générateur dépend aussi du type de traitement que l'on souhaite pratiquer. Par exemple, pour un débridement parodontal, la puissance/amplitude requise est nettement inférieure à celle nécessaire pour un détartrage. De la même façon, le type d'insert utilisé pour le débridement parodontal est différent de celui utilisé pour le détartrage. Par conséquent, pour chaque type de traitement dentaire, il existe une ou plusieurs familles d'inserts qui sont destinés à fonctionner dans une plage déterminée de puissance et d'amplitude des ondes ultrasonores.

II existe actuellement des appareils de traitement dentaire qui comprennent un générateur d'ultrasons dont la puissance peut être réglée en fonction du traitement pratiqué et de l'insert utilisée. Afin de faciliter l'usage de tels appareils pour les praticiens, les générateurs d'ultrasons sont équipés de touches permettant de sélectionner automatiquement la plage de puissance adaptée au traitement. Ces touches sont repérées par un code de couleur ou équivalent qui permet au praticien de sélectionner la plage de puissance adaptée.

Toutefois, comme expliqué plus haut, on utilise aussi des inserts spécifiques pour chaque traitement qui sont destinés à fonctionner dans une des plages de puissance préréglées sur l'appareil. Par conséquent, le praticien doit également contrôler que l'insert placé sur la pièce à main est bien adapté à la plage de puissance sélectionnée ou, inversement, sélectionner la plage de puissance correspondant à l'insert monté sur la pièce à main. A cet effet, une des solutions connues consiste à conditionner chaque insert sur un élément de support distinctif. L'élément support comprend un marquage en correspondance avec celui des touches de sélection des plages de puissances. Par exemple, si les touches sont repérées avec un code de couleur, les supports présentent chacun un code de couleur correspondant à celui de la touche permettant de sélectionner la plage de puissance optimale pour l'insert placé sur ce support

Cependant, cette solution présente encore certains inconvénients. En effet, une fois placé sur la pièce à main, l'insert est séparé de son support et, par conséquent, du moyen permettant le repérage de la plage de puissance adaptée. Ainsi, lors du traitement, il devient difficile pour le praticien de s'assurer que la plage de puissance sélectionnée est bien adaptée à l'insert présent sur la pièce à main. En outre, comme le support et l'insert sont séparables, il existe des risques de placer un insert sur un mauvais support, c'est-à-dire sur un support identifiant une plage de puissance qui n'est pas adaptée à l'insert ou inversement.

Une autre solution consiste à marquer l'insert en collant une étiquette ou en appliquant une peinture vernie sur celui-ci. Ce type de solution n'est toutefois pas satisfaisant en raison des conditions sévères auxquelles sont soumis les inserts. En effet, en fonctionnement, l'insert vibre à des fréquences de plusieurs dizaines de kilohertz sur des amplitudes de plusieurs centaines de micromètres et ce, dans des environnements humides. En outre, avant chaque utilisation, l'insert doit être systématiquement stérilisé dans des appareils de stérilisation autoclaves qui génèrent des températures autour de 130 °C. Le marquage de l'insert ne résiste pas à de telles conditions d'utilisation et disparaît très rapidement.

### Objet et résumé de l'invention

La présente invention a pour but de proposer une solution pour le repérage des inserts qui resiste aux conditions d'utilisation (vibrations, températures,...) de tels instruments de manière à fournir un moyen de contrôle simple et fiable de la plage de puissance et d'amplitude dans laquelle l'insert peut être utilisé.

Ce but est atteint grâce à un insert ultrasonique qui, conformément à la présente invention, comporte au moins une cavité formant un logement pour un élément de repérage, l'élément étant formé d'un matériau élastique qui présente une température de fusion supérieure à 130 °C.

Ainsi, l'insert selon l'invention présente une conception qui assure un marquage permanent de celui-ci puisqu'elle intègre un moyen d'identification dans la structure même de l'insert En effet, la cavité formée dans l'insert sert de logement pour l'élément de repérage et empêche ce dernier de sortir de l'insert. L'élément de repérage est réalisé en un matériau élastique, ce qui permet d'assurer une bonne tenue vis-à-vis des vibrations de l'insert. En outre le matériau de l'élément de repérage a une température de fusion supérieure à 130 °C afin de résister aux températures de stérilisation.

Un tel matériau peut être notamment du polytétrafluoréthylène (PTFE) ou un élastomère.

Selon un mode de réalisation de l'invention, la cavité et une gorge annulaire et l'élément de repérage un anneau qui vient se loger dans cette gorge.

La cavité peut en outre présenter des formes variées telles qu'une forme circulaire ou oblongue, l'élément de repérage présentant alors une forme adaptée à celle de la cavité.

Selon un mode de réalisation de l'invention, l'élément de repérage présente une couleur correspondant à une plage de puissance et d'amplitude des ondes ultrasonores dans laquelle l'insert est destiné à fonctionner. Cette couleur peut être obtenue notamment en incorporant une charge de pigments dans le matériau de l'anneau.

La ou les cavités sont placées de préférence à une distance déterminée de l'extrémité inférieure de l'insert de manière à dépasser, au moins partiellement, de la pièce à main lorsque l'insert est monté sur celle-ci. Ainsi, l'élément de repérage logé dans la cavité reste visible même lorsque l'insert est monté sur la pièce à main.

L'invention a également pour objet un appareil de traitement dentaire à ultrasons comprenant au moins une pièce à main chirurgicale reliée à un générateur d'ultrasons comportant des moyens pour sélectionner des plages de puissance et d'amplitude des ondes ultrasonores, l'appareil comprenant en outre au moins un insert tel que décrit précédemment.

Les moyens de sélection du générateur d'ultrasons peuvent être des touches ou équivalents présentant chacune une couleur ou un motif distinct correspondant à une plage de puissance et d'amplitude déterminée.

### Brève description des dessins

D'autres caractéristiques et avantages de l'invention ressortiront de la description suivante de modes particuliers de réalisation de l'invention, donnés à titre d'exemples non limitatifs, en référence aux dessins annexés, sur lesquels :
- la figure 1 est une vue en perspective d'un appareil de chirurgie dentaire à ultrasons conformément à un mode de réalisation de l'invention,
- la figure 2 est une vue en perspective d'un insert avant montage conformément à un mode de réalisation de l'invention,
- la figure 3 est une vue partiellement en coupe d'un insert selon un mode de réalisation de l'invention, l'insert étant monté sur une pièce à main,
- la figure 4 est une vue en perspective de quatre variantes de réalisation de l'insert des figures 2 et 3,
- la figure 5 est une vue en perspective d'un insert conformément à un autre mode de réalisation de l'invention, et
- la figure 6 est une vue en perspective d'un insert conformément à encore un autre mode de réalisation de l'invention.

### Description détaillée des modes de réalisation de l'invention

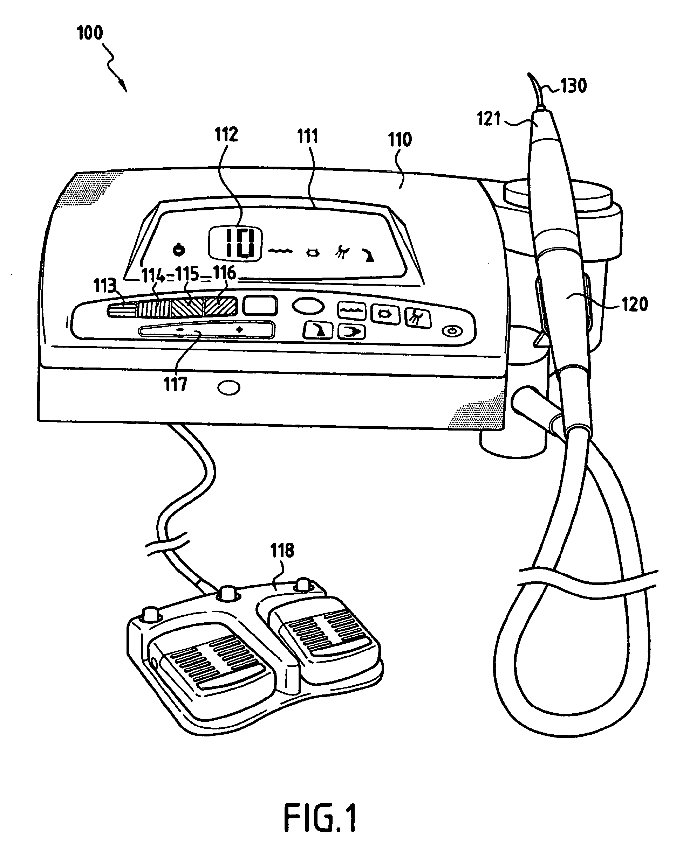

La figure 1 illustre un appareil de traitement à ultrasons 100 qui comprend un générateur d'ultrasons 110 relié à une pièce à main 120 équipé d'un insert 130. De façon bien connue, la pièce à main 120 comprend un transducteur (non représenté) formé par exemple d'un matériau piézoélectrique et couplé mécaniquement à l'insert 130 de manière à transmettre à ce dernier des ondes vibratoires dont l'amplitude est déterminée en fonction de la puissance fournie par le générateur d'ultrasons 110.

Le générateur comprend des moyens d'affichage 111 et une série de touches 113 à 116 correspondant chacune à une plage de puissance déterminée. A titre d'exemple, la touche 113, repérée sur la figure 1 par des hachures horizontales, correspond à la configuration de puissance et d'amplitude la plus faible recommandée par exemple pour les traitements délicats sur des surfaces fragiles avec des inserts très fins. La touche 114, repérée sur la figure 1 par des hachures verticales, correspond à une plage de puissance et d'amplitude moyennes adaptée pour des applications d'endodontie utilisant des inserts de formes minces et allongées. La touche 115, repérée sur la figure 1 par des hachures inclinées vers la droite, correspond à des niveaux de puissance et d'amplitude élevés adaptés aux traitements prophylactiques tels que le détartrage. La touche 116, repérée sur la figure par des hachures inclinées vers la gauche, permet de sélectionner la plage maximum de puissance et d'amplitude nécessaire par exemple en chirurgie apicale. Sur la figure 1, les touches 113 à 116 illustrées sur la figure 1 sont identifiées par un motif (hachures) différent pour chacune des touches. Toutefois, tout autre moyen de reconnaissance peut être utilisé comme par exemple une couleur spécifique pour chaque touche.

Ainsi, le générateur 110 comprend des moyens pour permettre une sélection de la plage de puissance optimale pour différentes applications cliniques. Une fois sélectionnée, la plage de puissance peut être reconnue par éclairage de la touche correspondante ou par un affichage particulier sur le générateur, comme par exemple un écran 112 qui affiche le motif ou la couleur de la touche sur laquelle le praticien a appuyée. Dans la plage de puissance sélectionnée, la puissance peut être réglée à l'aide d'une touche de réglage 117 ou au moyen d'un pédalier 118.

Pour identifier les inserts en fonction des plages de puissance et d'amplitude sélectionnables sur le générateur 110, la présente invention propose d'intégrer un marquage dans la structure même de l'insert.

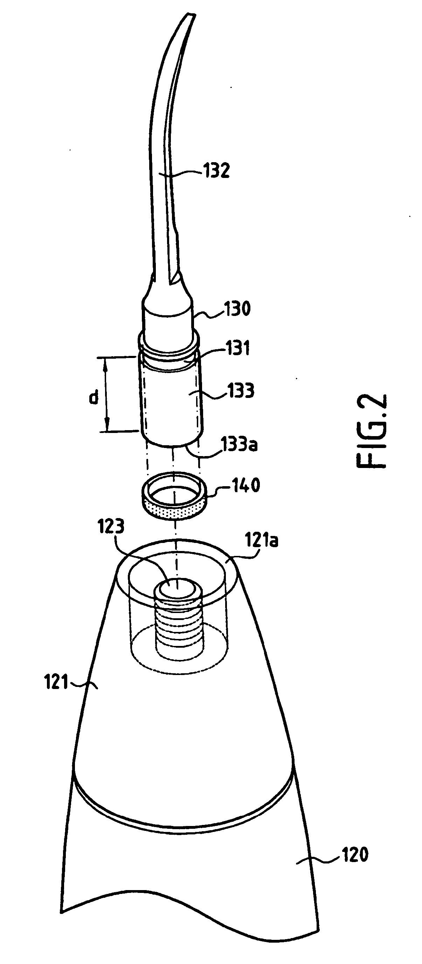

La figure 2 illustre un insert 130 conformément à un premier mode de réalisation de la présente invention. L'insert selon ce mode de réalisation comporte une gorge annulaire 131 qui forme un logement pour un anneau 140 constituant l'élément de repérage de l'insert.

L'anneau 140 est réalisé en un matériau qui est à la fois capable de supporter les vibrations ultrasoniques des inserts et de résister aux températures élevées des stérilisateurs autoclaves tout en étant biocompatible puisque l'insert est destiné à être utilisé pour des soins ou des actes chirurgicaux dans la bouche des patients. Tout type de matériau réunissant au moins ces trois propriétés est susceptible de convenir pour la réalisation de l'élément de repérage, ici l'anneau 140. Un tel matériau peut être du polytétrafluoréthylène (PTFE) qui est un matériau particulièrement bien adapté à l'utilisation envisagée dans la présente invention. En effet, le PTFE présente un allongement à la rupture (entre 250 et 450 %), un module d'élasticité (environ 7500 kg/cm²) et une résistance au choc (minimum 16 kg.cm/crn) très performants qui garantissent une bonne résistance vis-à-vis des vibrations ultrasonores de l'insert. De plus, le PTFE a des propriétés thermiques remarquables puisque sa température de fusion est de 327 °C, ce qui est bien supérieur aux températures rencontrées dans les stérilisateurs autoclaves (autour de 130 °C). II présente en outre un coefficient de dilatation thermique suffisamment faible (de l'ordre de 10.10⁵ pour des températures comprises entre 23 et 60 °C, et de 21.10⁵ pour des températures comprises entre 100 et 200 °C) qui l'empêche de se dilater sous l'effet des températures de stérilisation. Enfin, le PTFE est un matériau étanche qui est chimiquement et physiologiquement inerte et, par conséquent, biocompatible pour des applications médicales. D'autres matériaux présentant des propriétés similaires peuvent bien évidemment être utilisés pour former l'anneau 140. Parmi ces matériaux, on citera les élastomères telle que la silicone.

Pour permettre une identification visuelle de l'insert, telle qu'une reconnaissance par motif ou par couleur, le matériau constitutif de l'anneau 140 est soumis à un traitement supplémentaire qui vise à intégrer dans celui-ci le motif ou la couleur identique à celui présent sur la touche du générateur qui correspond à la plage de puissance adaptée. Pour une identification au moyen de codes de couleurs, le matériau de l'anneau est teinté à l'aide de colorants ou pigments directement incorporés dans le matériau. Ainsi, la couleur est présente dans tout le matériau et pas seulement en surface, ce qui permet de garantir un marquage permanent de l'insert même en cas d'usure ou d'endommagement de la surface de l'anneau exposée. Dans le cas d'un anneau en PTFE par exemple, on mélange le PTFE avec une charge de pigments afin de donner à l'anneau la couleur déterminée.

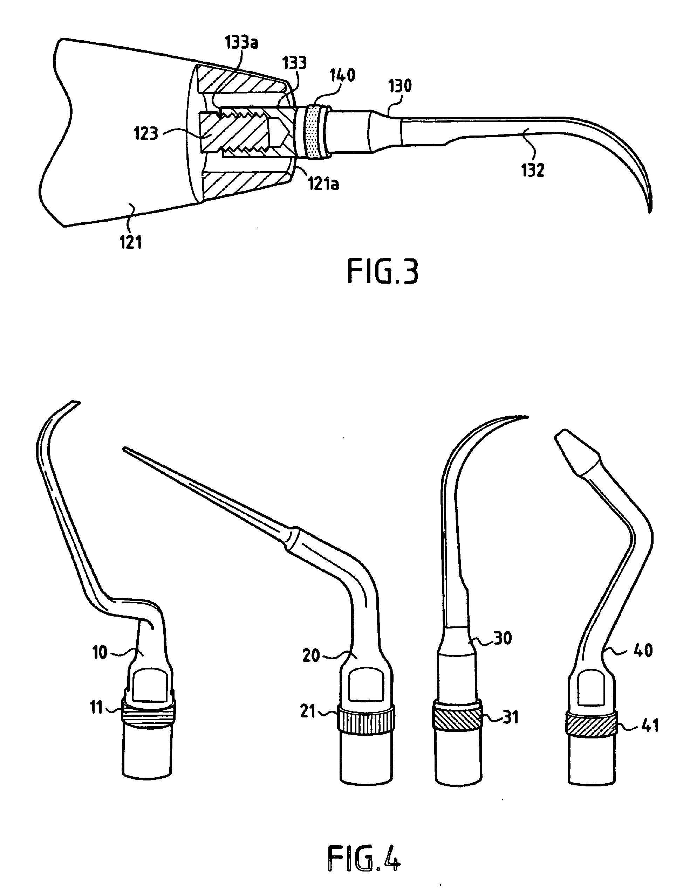

Comme illustrée sur les figures 2 et 3, l'insert est vissé sur un élément 123 solidaire du transducteur (non représenté) de la pièce à main 120. L'élément 123 est entouré par un manchon 121 qui recouvre une partie de la base 133 de l'insert lorsque celle-ci est montée sur la pièce à main. La gorge 131 est formée de préférence à une distance d de l'extrémité inférieure 133a de l'insert 130 qui permet à l'anneau de dépasser du bord 121a du manchon afin de rester visible même lorsque l'insert est monté sur la pièce à main comme le montre la figure 3.

Ainsi, conformément à l'invention, l'identification de la plage de puissance et d'amplitude dans laquelle l'insert doit être utilisé est clairement identifiable grâce à l'anneau qui est intégré dans la structure de l'insert. La figure 4 illustre quatre inserts 10, 20, 30 et 40, chacun d'entre eux étant destiné à fonctionner dans une des quatre plages de puissance et d'amplitude disponibles sur le générateur d'ultrasons 100 de la figure 1. Comme on peut le voir sur la figure 4, l'insert 10 comporte une bague 11 présentant un motif similaire à celui de la touche 113 du générateur 100 de la figure 1. Le praticien peut donc facilement identifier la plage de puissance et d'amplitude dans laquelle l'insert peut être utilisé. De même, les inserts 20, 30 et 40, qui ont chacun une plage de puissance et d'amplitude de fonctionnement propre, comportent des bagues 21, 31 et 41 présentant respectivement des motifs identiques à ceux des touches 114, 115 et 116. De la même manière, dans le cas d'une identification par codes de couleurs, les bagues 11, 21, 31 et 41 ont chacune une couleur différente qui correspond à celle des touches associées sur le générateur.

La solution mise en oeuvre pour le marquage de l'insert selon l'invention n'est pas limitée à l'utilisation d'un anneau, en tant qu'élément de repérage, logé dans une gorge circulaire comme décrit précédemment. En effet, l'insert peut comporter une ou plusieurs cavités de formes diverses dans lesquelles sont logées des éléments de repérage formées du même matériau que celui utilisé pour l'anneau 140.

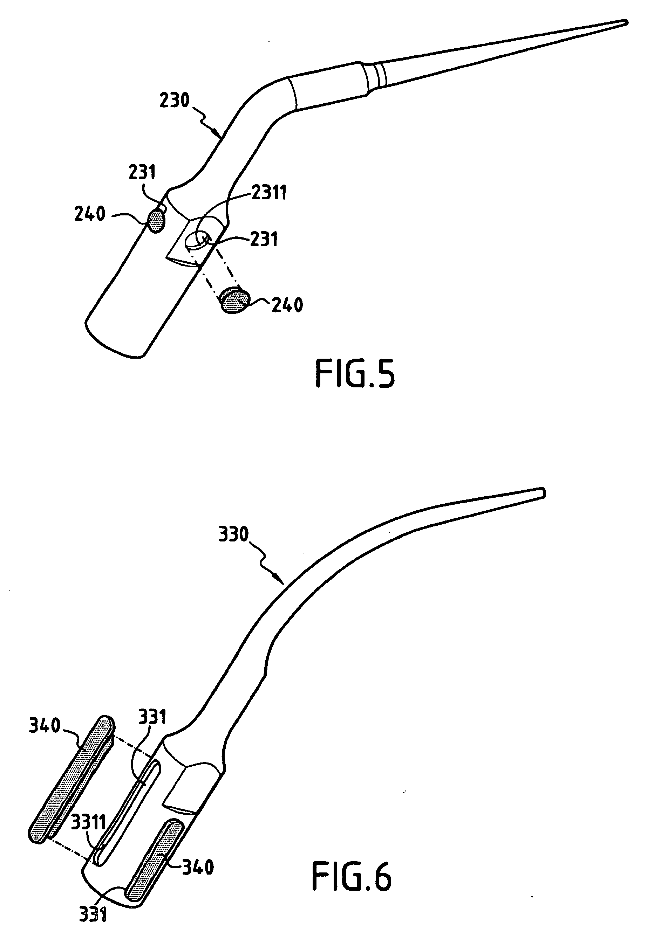

La figure 5 illustre un insert 230 suivant un autre mode de réalisation de l'invention. A la différence des inserts représentés en figures 3 et 4, l'insert 230 ne comporte pas de gorge annulaire mais des cavités 231 qui vont servir de logement pour des éléments de repérage en forme de pastilles 240 formées, comme pour l'anneau 140, en un matériau élastique ayant une température de fusion supérieure à 130 °C. Ce matériau peut être notamment du PTFE soumis à un traitement supplémentaire (ex. mélange PTFE avec une charge de pigments) pour y intégrer le motif ou la couleur identique à celui présent sur la touche du générateur qui correspond à la plage de puissance et d'amplitude adaptée.

Les cavités 231 sont ménagées sur l'insert 231 à une distance suffisante de la base de l'insert pour rester visibles une fois l'insert monté sur la pièce à main.

Selon encore un autre exemple de réalisation d'un insert à identification visuel conformément à l'invention, les cavités ainsi que les éléments de repérage permettant l'identification visuelle de la plage de puissance et d'amplitude de fonctionnement de l'insert peuvent présenter des formes oblongues. Comme illustré sur la figure 6, un insert 330 comporte des cavités 331 de forme oblongue qui servent de logements pour des éléments de repérage 340 également de forme oblongue. Les cavités 331 s'étendent à une distance suffisante de la base de l'insert pour rester visible même lorsque l'insert est monté sur la pièce à main.

De manière à assurer un bon maintien des pièces en matériau élastique, les cavités usinées dans l'insert pour ménager des logements pour les éléments de repérage peuvent présenter des dimensions en surface inférieures à celle du fond de la cavité (ex. cavité en forme de trapèze) de manière à former un rétrécissement en surface de l'insert. Sur les figures 5 et 6, les bords 2311 et 3311 respectivement des cavités 231 et 331 peuvent être inclinés vers le fond des cavités formant une ouverture en surface de l'insert de dimensions inférieures à celles du fond des cavités. Dans ce cas, la pièce en matériau élastique ou la portion de celle-ci destinée à être introduite dans la cavité présente alors des dimensions proches de celles du fond de la cavité pour être rentrée en force dans cette dernière. Ainsi, sans un effort de traction important sur la pièce, cette dernière ne peut s'échapper de la cavité.

Le maintien des éléments de repérage peut encore être renforcé en collant ces éléments dans les cavités. Dans le cas du PTFE ou de la silicone par exemple qui sont des matériaux dits « basse énergie de surface », on utilise un adhésif adapté tel qu'une colle acrylique.

Les inserts de l'invention peuvent être utilisés avec des appareils périphériques à usage dentaire tels que l'appareil de traitement à ultrasons de la figure 1 qui, de par son ergonomie et ses fonctionnalités, constitue un produit fini. Ces inserts peuvent être également mis en oeuvre avec des appareils présentés sous forme de modules destinés à être intégrés (technologie OEM) avec d'autres modules dans des produits dédiés tels que les postes de travail pour cabinet dentaire.

## Revendications

1. Insert ultrasonique ou sonotrode (130 ; 230 ;330) comportant une extrémité distale libre et une extrémité proximale (133a) destinée à être vissée sur un élément solidaire d'un transducteur d'une pièce à main chirurgicale (120), **caractérisé en ce qu'**il comporte au moins une cavité (131 ; 231 ; 331) formant un logement pour un élément de repérage (140 ; 240 ; 340) et **en ce que** l'élément de repérage est formé d'un matériau élastique qui présente une température de fusion supérieure à 130 °C.

2. Insert selon la revendication 1, **caractérisé en ce que** la cavité est une gorge annulaire (131) et **en ce que** l'élément de repérage est un anneau (140) logé dans ladite gorge annulaire.

3. Insert selon la revendication 1, **caractérisé en ce que** la cavité (231) est de forme circulaire et **en ce que** l'élément de repérage est une pastilie (240) logée dans ladite cavité.

4. Insert selon la revendication 1, **caractérisé en ce que** la cavité (331) est de forme oblongue et **en ce que** l'élément de repérage est une pièce de forme oblongue (340) logée dans ladite cavité.

5. Insert selon l'une quelconque des revendications 1 à 4, **caractérisé en ce que** la cavité présente des bords inclinés vers le fond de la cavité de manière à former un rétrécissement en surface de l'insert.

6. Insert selon l'une quelconque des revendications 1 à 5, **caractérisé en ce que** le matériau de l'élément de repérage (140 240 ; 340) est biocompatible.

7. Insert selon l'une quelconque des revendications 1 à 6, **caractérisé en ce que** l'élément de repérage (140 ; 240 ; 340) est en potytétraftuoréthytène (PTFE).

8. Insert selon l'une quelconque des revendications 1 à 6, **caractérisé en ce que** l'élément de repérage (140 ; 240 ; 340) est en élastomère.

9. Insert selon l'une quelconque des revendications 1 à 8, **carectérisé en ce que** l'élément de repérage (140 ; 240 ; 340) présente une codeur correspondant à une plage de puissance et d'amplitude des ondes ultrasonores dans laquelle l'insert est destiné à fonctionner.

10. Insert selon la revendication 9, **caractérisé en ce que** le matériau de l'élément de repérage (140 ; 240 ; 340) comprend une charge de pigments de manière à conférer à l'élément ladite couleur correspondante.

11. Insert selon l'une quelconque des revendications 1 à 10, **caractérisé en ce que** la cavité (131) est placée à une distance (d) de l'extrémité inférieure (133a) de l'insert (130) de manière à dépasser de la pièce à main (120) lorsque l'insert est monté sur celle-ci.

12. Appareil de traitement dentaire à ultrasons (100) comprenant au moins une pièce à main chirurgicale (120) reliée à un générateur d'ultrasons (110) comportant des moyens pour sélectionner des plages de puissance et d'amplitude des ondes ultrasonores, **caractérisé en ce qu'**il comprend en outre au moins un insert (130 ; 230 ; 330) selon l'une quelconque des revendication 1 à 11,

13. Appareil salon la revendication 12, **caractérisé en ce que** les moyens pour sélectionner des plages de puissance et d'amplitude des ondes ultrasonores comprennent des touches de sélection (113, 114, 115, 116), chaque touche présentant une couleur ou un motif distinct correspondant à une plage de puissance et d'amplitude déterminée.

## Claims

1. An ultrasound insert (130; 230; 330) comprising a top free end and a bottom end (133a) intended to be screwed to a transducer of a surgical handpiece (120), the insert being **characterized in that** it includes at least one cavity (131; 231; 331) forming a housing for an identifier element (140; 240; 340), and **in that** the identifier element is made of an elastic material that presents a melting temperature higher than 130°C.

2. An insert according to claim 1, **characterized in that** the cavity is an annular groove (131) and **in that** the identifier element is a ring (140) housed in said annular groove.

3. An insert according to claim 1, **characterized in that** the cavity (231) is circular in shape and **in that** the identifier element is a pellet (240) housed in said cavity.

4. An insert according to claim 1, **characterized in that** the cavity (331) is oblong in shape and **in that** the identifier element is a piece of oblong shape (340) housed in said cavity.

5. An insert according to any one of claims 1 to 4, **characterized in that** the cavity p:resents edges that slope towards the bottom of the cavity in such a manner as to form a constriction at the surface of the insert.

6. An insert according to any one: of claims 1 to 5, **characterized in that** the material of the identifier element (140; 240; 340) is biocompatible.

7. An insert according to any one of claims 1 to 6, **characterized in that** the identifier element (140; 240; 340) is made of polytetrafluoroethylene (PTFE).

8. An insert according to any one of claims 1 to 6, **characterized in that** the identifier element (140; 240; 340) is made of elastomer.

9. An insert according to any one of claims 1 to 8, **characterized in that** the identifier element (140; 240; 340) presents a color corresponding to an ultrasonic wave power and amplitude range in which the insert is designed to operate.

10. An insert according to claim 9, **characterized in that** the material of the identifier element (140; 240; 340) includes a pigment filler so as to confer said corresponding color to the element.

11. An insert according to any one of claims 1 to 10, **characterized in that** the cavity (131) is placed at a distance (d) from the bottom end (133a) of the insert (130) so as to project beyond the handpiece (120) when the insert is mounted thereon.

12. An ultrasonic dental sealer appliance (100) comprising at least one surgical handpiece (120) connected to an ultrasound generator (110) including means for selecting ultrasonic wave power and amplitude ranges, the appliance being **characterized in that** it further comprises at least one insert (130; 230; 330) according to any one of claims 1 to 11.

13. An appliance according to claim 12, **characterized in that** the means for selecting the ultrasonic wave power and amplitude ranges comprise selection keys (113, 114, 115, 116), each key presenting a distinct color or pattern corresponding to a determined power and amplitude range.

## Patentansprüche

1. Ultraschalleinsatz oder Sonotrode (130; 230; 330) mit einem freien distalen Ende und einem proximalen Ende (133a), welches dazu bestimmt ist, auf ein Element, das mit einem Wandler eines chirurgischen Handstücks (120) fest verbunden ist, aufgeschraubt zu werden, **dadurch gekennzeichnet, daß** er wenigstens einen Hohlraum (131; 231; 331) umfaßt, der eine Aufnahme für ein Kennzeichnungselement (140; 240; 340) bildet, und daß das Kennzeichnungselement aus einem elastischen Material ausgebildet ist, das eine Schmelztemperatur von über 130 °C aufweist.

2. Einsatz nach Anspruch 1, **dadurch gekennzeichnet, daß** der Hohlraum eine ringförmige Nut (131) ist und daß das Kennzeichnungselement ein in der ringförmigen Nut aufgenommener Ring (140) ist.

3. Einsatz nach Anspruch 1, **dadurch gekennzeichnet, daß** der Hohlraum (231) kreisförmig ausgebildet ist und daß das Kennzeichnungselement ein in dem Hohlraum aufgenommenes Plättchen (240) ist.

4. Einsatz nach Anspruch 1, **dadurch gekennzeichnet, daß** der Hohlraum (331) eine längliche Form aufweist und daß das Kennzeichnungselement ein in dem Hohlraum aufgenommenes Teil länglicher Form (340) ist.

5. Einsatz nach einem der Ansprüche 1 bis 4, **dadurch gekennzeichnet, daß** der Hohlraum zum Grund des Hohlraums geneigte Ränder aufweist, so daß eine Verjüngung an der Oberfläche des Einsatzes gebildet wird.

6. Einsatz nach einem der Ansprüche 1 bis 5, **dadurch gekennzeichnet, daß** das Material des Kennzeichnungselements (140; 240; 340) biokompatibel ist.

7. Einsatz nach einem der Ansprüche 1 bis 6, **dadurch gekennzeichnet, daß** das Kennzeichnungselement (140; 240; 340) aus Polytetrafluorethylen (PTFE) besteht.

8. Einsatz nach einem der Ansprüche 1 bis 6, **dadurch gekennzeichnet, daß** das Kennzeichnungselement (140; 240; 340) aus Elastomer besteht.

9. Einsatz nach einem der Ansprüche 1 bis 8, **dadurch gekennzeichnet, daß** das Kennzeichnungselement (140; 240; 340) eine Farbe aufweist, die einem Leistungs-und Amplitudenbereich der Ultraschallwellen, in dem der Einsatz zu arbeiten bestimmt ist, entspricht.

10. Einsatz nach Anspruch 9, **dadurch gekennzeichnet, daß** das Material des Kennzeichnungselements (140; 240; 340) einen Pigmentfüllstoff umfaßt, um dem Element die entsprechende Farbe zu verleihen.

11. Einsatz nach einem der Ansprüche 1 bis 10, **dadurch gekennzeichnet, daß** der Hohlraum (131) in einem Abstand (d) von dem unteren Ende (133a) des Einsatzes (130) plaziert ist, so daß er über das Handstück (120) hinausragt, wenn der Einsatz auf diesem angebracht ist.

12. Gerät zur Zahnbehandlung mittels Ultraschall (100), umfassend wenigstens ein chirurgisches Handstück (120), das mit einem Ultraschallgenerator (110) verbunden ist, der Mittel zum Auswählen von Leistungs- und Amplitudenbereichen der Ultraschallwellen aufweist, **dadurch gekennzeichnet, daß** es ferner wenigstens einen Einsatz (130; 230; 330) nach einem der Ansprüche 1 bis 11 umfaßt.

13. Gerät nach Anspruch 12, **dadurch gekennzeichnet, daß** die Mittel zum Auswählen von Leistungs- und Amplitudenbereichen der Ultraschallwellen Auswahltasten (113, 114, 115, 116) umfassen, wobei jede Taste ein(e) unterschiedliche(s) Farbe oder Muster aufweist, die bzw. das einem bestimmten Leistungs- und Amplitudenbereich entspricht.
